(19) Europäisches Patentamt
European Patent Office
Office européen des brevets

(11) Veröffentlichungsnummer: **0 429 153 A1**

(12) **EUROPÄISCHE PATENTANMELDUNG**

(21) Anmeldenummer: **90250287.1**

(51) Int. Cl.5: **C07D 457/02**, A61K 31/48

(22) Anmeldetag: **20.11.90**

(30) Priorität: **20.11.89 DE 3938701**

(43) Veröffentlichungstag der Anmeldung: **29.05.91 Patentblatt 91/22**

(84) Benannte Vertragsstaaten: **AT BE CH DE DK ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**W-1000 Berlin 65(DE)**

(72) Erfinder: **Sauer, Gerhard, Dr.**
**Königsbacher Zeile 41A**
**W-1000 Berlin 28(DE)**
Erfinder: **Brumby, Thomas, Dr.**
**Hauptstrasse 13**
**W-1000 Berlin 30(DE)**
Erfinder: **Wachtel, Helmut, Dr.**
**Suarezstrasse 22**
**W-1000 Berlin 19(DE)**
Erfinder: **Löschmann, Peter-Andreas**
**Württembergallee 8**
**W-1000 Berlin 19(DE)**
Erfinder: **Turner, Jonathan, Dr.**
**Ortwinstrasse 7**
**W-1000 Berlin 28(DE)**

(54) **Neue Agroclavin- und Elymoclavin-Derivate, Verfahren zu deren Herstellung und deren Verwendung in Arzneimitteln.**

(57) Es werden Ergolin-Derivate der Formel I und deren Säureadditionssalze

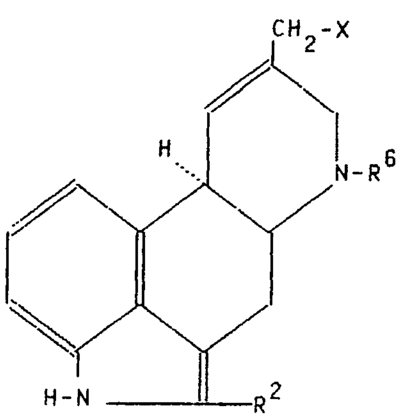

I

worin $R^2$, $R^6$ und X die in der Anmeldung genannte Bedeuting haben, beschrieben sowie die Herstellung derselben und Arzneimittel enthaltend diese Verbindungen.

EP 0 429 153 A1

Die Erfindung betrifft neue Agroclavin- und Elymoclavin-Derivate, deren Herstellung und Verwendung in Arzneimitteln sowie Zwischenverbindungen zur Herstellung derselben und das Verfahren zur Herstellung der Zwischenverbindungen.

Agroclavin und Elymoclavin sind hochwirksame natürliche Ergotalkaloide, die auf Gräsern vorkommen, die von Claviceps purpurea infiziert sind. Sie zeigen das ganze Spektrum der von Ergotalkaloiden bekannten Wirkungen.

Durch Einführung von längerkettigen Kohlenwasserstoffen in 6-Stellung und durch Substitution in 2-Stellung wird die Beeinflussung von verschiedenen Rezeptoren verändert und die metabolische Stabilität erhöht. Die erfindungsgemäß substituierten Ergolinderivate weisen daher im Vergleich mit den 6-Methyl-substituierten Agroclavin- und Elymoclavin-Derivaten eine verstärkte dopaminerge Wirksamkeit und größere Selektivität auf. Gleichzeitig wird der metabolische Abbau verzögert.

Die Erfindung betrifft Verbindungen der Formel I und deren Säureadditionssalze

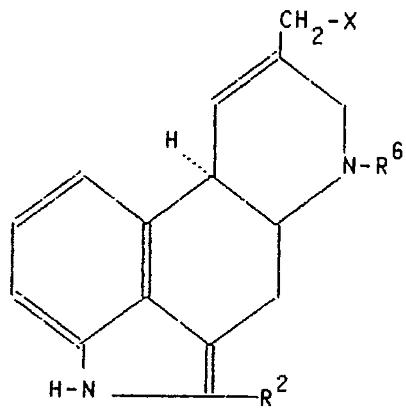

I

worin

$R^2$ Halogen, $C_{1-4}$-Alkyl, $C_{2-4}$-Alkenyl,

$R^6$ $C_{2-6}$-Alkyl, $C_{3-6}$-Alkenyl oder $C_{3-5}$-Cycloalkyl-$C_{1-2}$-Alkyl und

X Wasserstoff, Hydroxy oder $C_{1-6}$-Acyloxy ist.

Die physiologisch verträglichen Säureadditionssalze leiten sich von den bekannten anorganischen und organischen Säuren ab wie zum Beispiel Salzsäure, Schwefelsäure, Bromwasserstoffsäure, Zitronensäure, Maleinsäure, Fumarsäure, Weinsäure u.a..

Unter Alkyl ist jeweils ein geradkettiger oder verzweigter Alkylrest zu verstehen wie beispielsweise Methyl, Ethyl, n-Propyl, Isopropyl, n-Butyl, Isobutyl, sek. Butyl, tert. Butyl, Pentyl, Hexyl, 2,2-Dimethylpropyl, 2-Methylbutyl, Isopentyl u.a.

Bedeuten $R^2$ oder $R^6$ einen Alkenylrest, so enthält dieser bevorzugt nur eine Doppelbindung, wobei die Doppelbindung in $R^6$ nicht benachbart zum Stickstoffatom stehen kann. Als Alkenylreste sind beispielsweise geeignet: Vinyl, 1-Propenyl, 2-Propenyl, 1-Methyl-2-propenyl, 1-Butenyl, Methallyl.

Bedeutet $R^6$ eine Cycloalkyl-alkyl-Gruppe, so ist beispielsweise Cyclopropylmethyl, Cyclopropylethyl oder Cyclobutylmethyl gemeint.

Die Acylgruppen im Substituenten X leiten sich von aliphatischen Carbonsäuren ab wie Ameisensäure, Essigsäure, Propionsäure, Buttersäure, Capronsäure, Trimethylessigsäure, wobei bevorzugt Essigsäure verwendet wird. Bevorzugte Ausführungsformen für $R^6$ sind Alkyle und Alkenyle mit bis zu 4 Kohlenstoffatomen und Cycloalkyl-alkyle mit bis zu 5 Kohlenstoffatomen.

Die Verbindungen der Formel I können als E- oder Z-Isomere oder, falls ein chirales Zentrum im Rest $R^2$ vorhanden ist, als Diastereomere und als Gemische derselben auftreten. Die Isomeren und Isomerengemische sind von der vorliegenden Erfindung auch umfaßt.

Die Verbindungen der Formel I sowie deren Säureadditionssalze sind auf Grund ihrer zentral dopaminergen Wirksamkeit als Arzneimittel verwendbar. Da sie sich insbesondere durch dopaminagonistische Wirkung auszeichnen, ohne daß starke $\alpha$-adrenerge Effekte auftreten, eignen sich die erfindungsgemäßen Verbindungen insbesondere zur Behandlung des Morbus Parkinson.

Die dopaminagonistische Wirkung wurde mit Hilfe der von Horowski beschriebenen Methode der automatischen Registrierung von Stereotypien an Ratten bestimmt (Arzneim. Forsch. 12, 2281-2286, 1978): Unmittelbar nach intraperitonealer Prüfsubstanz- bzw. Vehikelverabreichung werden männliche Wistar-Ratten (90-120 g) einzeln in Zwangskäfige aus Acrylglas gesetzt. Uber ein vor dem Kopf der Tiere angebrachtes elektrodynamisches Aufnahmesystem wird die Zahl der Kontakte an einem stählernen Becher

mit einem zentralen Metallstab als Folge der stereotypen Kau-, Leck- und Nagebewegungen während 60 Minuten registriert. Die Mittelwerte ± S.E.M. der Anzahl der Kontakte während 60 Minuten für die verschiedenen Behandlungsgruppen, die mit jeweils 12 Tieren besetzt sind, werden berechnet und die Signifikanz der Unterschiede zwischen den Mittelwerten der verschiedenen Prüfsubstanzdosen im Vergleich zur Vehikel-behandelten Kontrollgruppe mit Hilfe der einfachen Varianzanalyse in Verbindung mit dem Dunnett-Test ermittelt.

Die Ergebnisse sind in der nachfolgenden Tabelle dargelegt.

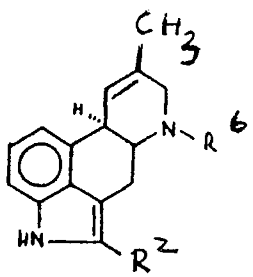

TABELLE

Auslösung von Stereotypien bei Ratten während 60 Minuten nach intraperitonealer Behandlung mit Vehikel bzw. verschiedenen Dosen von Ergolinen
(x:p<0.01, xx:p<0.01, Varianzanalyse/Dunnett-Test vs. Kontrolle)
n: Anzahl der Versuchstiere pro Behandlungsgruppe

| | | | | Stereotypien (counts pro 60 Minuten) Mittelwert ± S.F.M.) | | | | |
|---|---|---|---|---|---|---|---|---|
| | | | | Prüfsubstanzdosis | | | | |
| $R^2$ | $R^6$ | n | Kontrolle | 0.025 | 0.1 | 0.39 | 1.56 | 6.25 |
| $CH_3$ | $CH_2CH_2CH_3$ | 12 | 859 ± 98 | 4726 ± 1148xx | 7601 ± 1182xx | 9200 ± 672xx | 10150 ± 598xx | -- |
| Cl | $CH_2CH_2CH_3$ | 12 | 1195 ± 121 | 2091 ± 394 | 6470 ± 1018xx | 7364 ± 927xx | 8837 ± 699xx | 10397 ± 1126xx |

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Ole, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe wie Konservierungs-, Stabilisierungs-, Netzmittel oder Emulgatoren, Salze zur Veränderung des osmotischen Drucks oder Puffer.

Die erfindungsgemäßen Verbindungen werden in einer Dosis von 0,001 bis 10 mg aktiver Substanz in einen physiologisch verträglichen Träger eingebracht. Die Anwendung der erfindungsgemäßen Verbindungen erfolgt in einer Dosis von 0,00001 bis 0,1 mg/kg/Tag, vorzugsweise 0,001 bis 0,1 mg/kg/Tag analog dem bekannten Mittel Bromocryptin.

Die Herstellung der erfindungsgemäßen Verbindungen der Formel I kann nach an sich bekannten Methoden durchgeführt werden.

Beispielsweise gelangt man zu Verbindungen der Formel I, indem man

a) Verbindungen der Formel II

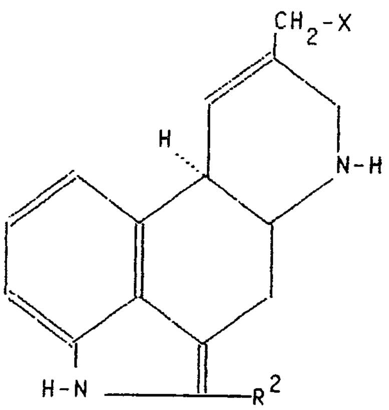

II

worin $R^2$ und X die obige Bedeutung haben, alkyliert oder alkenyliert zu Verbindungen mit $R^6$ in der obigen Bedeutung oder

b) Verbindungen der Formel III

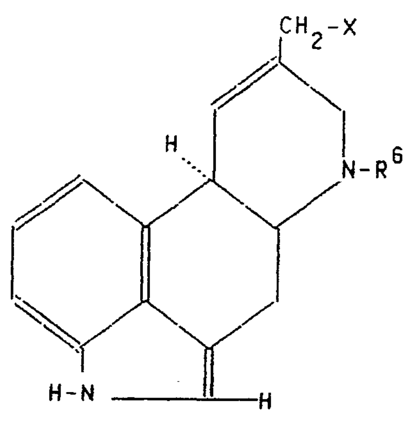

III

worin $R^6$ und x die obige Bedeutung haben, halogeniert zu Verbindungen der Formel I mit $R^2$ in der Bedeutung von Halogen oder

c) Verbindungen der Formel IV

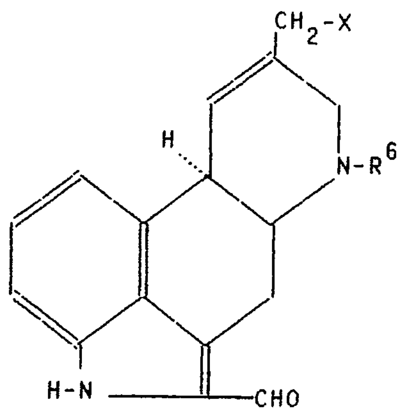

IV

worin $R^6$ und X die obige Bedeutung haben,

α) in einer Wittig-Reaktion zu Verbindungen mit $R^2$ in der Bedeutung eines $C_{2-4}$-Alkenylrestes umsetzt oder

β) in einen 1-Hydroxy-$C_{1-4}$-Alkylrest überführt und diesen gewünschtenfalls anschließend dehydratisiert zu Verbindungen mit $R^2$ in der Bedeutung eines $C_{2-4}$-Alkenylrestes und gewünschtenfalls anschließend die

nach α) oder β) erhaltenen Verbindungen, gegebenenfalls nach Veresterung des 2-$CH_2OH$-Restes, reduziert zu Verbindungen mit $R^2$ in der Bedeutung eines $C_{1-4}$-Alkylrestes und gewünschtenfalls anschließend die Acylgruppe in 8-Stellung abspaltet, die Isomeren trennt, und die Säureadditionssalze bildet.

Die Substitution in 6-Stellung nach dem Verfahren a) kann beispielsweise nach A. Cerny et al. Coll. Czech. Chem. Comm. 49, 2828 (1984) oder nach dem in der EP-21206 beschriebenen Verfahren durchgeführt werden, indem man die 6H-Verbindung der Formel II mit den entsprechenden $R^6$-Halogeniden (Bromiden, Chloriden, Iodiden) umsetzt. Zweckmäßigerweise erfolgt die Reaktion in einem inerten Lösungsmittel wie Dimethylsulfoxid, Dimethylformamid, Acetonitril oder Nitromethan in Gegenwart von Basen wie Alkalihydroxiden oder -carbonaten.

Die Halogenierung der Verbindungen der Formel III nach der Verfahrensvariante b) erfolgt nach den in EP-56358 bzw. EP-141387 beschriebenen Verfahren durch direkte Halogenierung der 2H-Verbindung mit geeigneten Halogenierungsmittel bzw. Brom in Gegenwart von Bromwasserstoff.

Die Einführung des Substituenten $R^2$ kann vor oder nach Substitution in 6-Stellung erfolgen.

Die Umwandlung der 2-Formyl-Verbindung der Formel IV zu Verbindungen der Formel I, worin $R^2$ einen Alkenylrest bedeutet, erfolgt nach Methode cα) in einer Wittigreaktion, indem beispielsweise mit Alkyltriphenylphosphoniumhalogenid in polaren Lösungsmitteln wie cyclischen und acyclischen Ethern, chlorierten Kohlenwasserstoffen, Dimethylformamid oder Dimethylsulfoxid bei Temperaturen von -50 °C bis + 50 °C umgesetzt wird, wobei zur Erzeugung des Ylens starke Basen wie Alkalialkoholate, Lithiumorganyl u.a. zugesetzt werden.

Verbindungen der Formel I mit $R^2$ in der Bedeutung eines Alkenylrestes können auch nach dem Verfahren cβ) hergestellt werden, indem man die Verbindungen der Formel IV durch Grignardierung oder Lithium-alkylierung in die 1-Hydroxyalkylierten-Derivate überführt und anschließend dehydratisiert. Die Grignardierung kann mit den üblichen Grignard-Reagenzen wie Alkylmagnesium-halogeniden in einem aprotischen Lösungsmittel wie cyclischen und acyclischen Ethern bei tiefen Temperaturen (-70 °C bis 20 °C) erfolgen. Die Umsetzung mit Alkyl-Lithium erfolgt unter analogen Bedingungen. Die anschließende Einführung der Doppelbindung kann mit den üblichen Dehydratisierungsmethoden vorgenommen werden wie beispielsweise mit Sulfonaten oder Acetaten wie Methansulfonsäurechlorid in polaren Lösungsmitteln wie Ethern in Gegenwart einer Base und gegebenenfalls unter Erhitzen.

Enthält der Substituent $R^2$ eine exocyclische Doppelbindung, so kann diese reduziert werden zum entsprechenden Alkylderivat. Beispielsweise kann die Reduktion mit Lithium oder Natrium in flüssigem Ammoniak in einem inerten Lösungsmittel wie cyclischen und acyclischen Ethern in Gegenwart eines Protonenspenders wie aliphatischen Alkoholen vorgenommen werden.

Auch die nach Verfahren cβ) erhaltenen 1-hydroxy-alkylierten Derivate können in üblicher Weise beispielsweise durch Umsetzung mit $NaBH_4$ in Essigsäure zum entsprechenden 2-Alkyl-Derivat reduziert werden.

Zur Einführung der 2-$CH_3$-Gruppe ist es vorteilhaft, vor der Reduktion des 2-$CH_2OH$-Restes diesen mit Säuren wie Pivalinsäure, Essigsäure, Benzoesäure zu verestern und anschließend nach den bekannten

Verfahren wie in der Deutschen Patentanmeldung P 3923211.5 beschrieben zu reduzieren.

Die selektive Abspaltung der Acylgruppe in 8-Stellung erfolgt nach den bekannten Verfahren zur Esterspaltung wie beispielsweise mit Alkalialkoholaten.

Zur Bildung von Salzen wird eine Verbindung der Formel I beispielsweise in wenig Methanol oder Methylenchlorid gelöst und mit einer konzentrierten Lösung der gewünschten Säure versetzt.

Die Isomerengemische können nach den üblichen Methoden wie beispielsweise Kristallisation, Chromatographie oder Salzbildung in die Diastereomeren bzw. E/Z-Isomeren aufgetrennt werden.

Die Erfindung umfaßt auch die Verbindungen der Formel IV′, die wertvolle Zwischenprodukte zur Herstellung pharmakologisch wirksamer Verbindungen darstellen. Die Umwandlung der Zwischenprodukte in die Wirksubstanzen erfolgt nach den vorne beschriebenen Methoden zur Einführung der gewünschten Substituenten in 8-, 6- oder 2-Stellung.

Soweit die Herstellung der Ausgangsverbindungen nicht beschrieben wird, sind diese bekannt oder analog zu bekannten Verbindungen oder hier beschriebenen Verfahren herstellbar.

Beispielsweise kann die Herstellung des 2-Formyl-Derivates der Formel IV′ nach Anspruch 6 durch Oxidation der Mannich Base oder der 2-Hydroxymethyl-Verbindung analog den im EP-A-89730164 beschriebenen Methoden erfolgen. Die Einführung der 2-Hydroxymethyl-Gruppe gelingt beispielsweise mit Paraformaldehyd/Dimethylaluminiumchlorid wie in der Deutschen Patentanmeldung P 3923211.5 beschrieben.

Die nachfolgenden Beispiele sollen das erfindungsgemäße Verfahren erläutern.

**Ausgangsverbindungen**

1.) **8,9-Didehydro-8-methyl-ergolin-6-carbonitril**

56 g (0,23 mol) Agroclavin wurden in 1,4 l Dioxan p.A. gelöst und 32,8 g (0,23 mol) Kaliumcarbonat zugegeben. 50 g (0,47 mol) Bromcyan (eine Flasche, Merck) wurden in 300 ml Dioxan gelöst und auf einmal zugegeben. Man spülte mit 100 ml Dioxan nach und ließ 2 Stunden bei Raumtemperatur rühren. Dann wurde der größte Teil des Lösungsmittel im Vakuum abdestilliert (80 °C Badtemperatur), wobei die Vorlage zur Vernichtung des überschüssigen Bromcyans mit KOH beschickt wurde. Der Rest des Dioxans wurde am Rotationsverdampfer entfernt; der Rückstand mit Dichlormethan extrahiert und eingeengt. Durch Kristallisation aus Methanol erhielt man in drei Fraktionen insgesamt 47,7 g (81 %) reines Produkt. Die verbleibenden 6,0 g Mutterlauge wurden chromatographiert (1 kg Kieselgel, Diisopropylether/Hexan 2:1, 3:1, 6:1, und Diisopropylether). Man erhielt weitere 3,6 g (6 %), Ausbeute: 87 % kristallin, $[\alpha]_D$ = + 53,5 ° (c = 1,0 in Chloroform), Schmelzpunkt 180-190 °C (Zers.).

2.) **8,9-Didehydro-8-hydroxymethyl-6-ergolin-carbonitril**

Die Darstellung erfolgte wie in vorstehenden Beispiel, Ausbeute 90 %, $[\alpha]_D$ = + 30 ° (0,5 % in Pyridin).

3.) **8,9-Didehydro-8-methyl-ergolin**

Zu 1,2 l wasserfreiem Ammoniak (vor der Kondensation über KOH getrocknet) wurden 37,9 g (0,15 mol) 8,9-Didehydro-8-methyl-ergolin-6-carbonitril in 900 ml Tetrahydrofuran gelöst zugegeben. Man ließ 10 Minuten rühren, um die Temperatur wieder auf - 78 °C sinken zu lassen und gab dann insgesamt 18 g (2,2 mol) Kalium in kleinen Stücken zu. Danach war die Lösung rot gefärbt. Die Zugabe einiger ml Methanol stellte die gewohnte Blaufärbung her, die man nach 10 Minuten durch Zugabe von ingesamt 150 ml Methanol zerstörte. Nach Abdampfen des Ammoniak wurde das Lösungsmittel im Vakuum entfernt und der Rückstand gründlich mit Dichlormethan extrahiert. Der Extrakt wurde mit Wasser und gesättigter NaClLösung gewaschen und nach Trocknung über Natriumsulfat eingedampft. Der Rückstand wurde aus Methanol kristallisiert, wobei 22,2 g (65 %) reines Produkt in drei Fraktionen erhalten wurde, Die Chromatographie der Mutterlauge liefert weitere 3,8 g, Ausbeute: 76 % kristallisiert, $[\alpha]_D$ = - 112,8 ° (c = 1,0 Chloroform), Schmelzpunkt 125-133 °C.

4.) **8,9-Didehydro-8-hydroxymethyl-ergolin**

In etwa 2 Liter getrocknetem, flüssigen Ammoniak suspendiert man bei - 78 °C 37 g Lithiumamid (1,6 Mol) und gibt dann 54 g 8,9-Didehydro-8-hydroxymethyl-6-ergolin-carbonitril (0,2 Mol) in fester Form zu. Nach 30 Minuten versetzt man mit 7,5 g geschnittenem Lithium, wobei nach 30 Minuten eine intensive

Blaufärbung auftrat. 5 Minuten später entfärbt man die Mischung durch Zugabe von Ammoniumchlorid. Man gibt 50 ml Wasser zu, dampft den Ammoniak ab und versetzt vorsichtig mit insgesamt 1,5 l Wasser. Die Mischung wird 30 Minuten unter Eiskühlung gerührt, die Kristalle abgesaugt, gewaschen und getrocknet, Ausbeute 40,6 g (83 % der Theorie), $[\alpha]_D$ = - 99 ° (0,5 % in Pyridin).

5.) **8,9-Didehydro-8-hydroxymethyl-2-methyl-ergolin**

A) 8-Acetoxymethyl-8,9-didehydro-2-hydroxymethyl-6-ergolin-carbonitril

Man löst 44,4 g 8-Acetoxymethyl-8,9-didehydro-6-ergolin-carbonitril (144 mmol) in 7,5 Liter Dichlormethan, gibt 65 g Paraformaldehyd (2,1 Mol) zu und kühlt die Mischung auf - 50 °C ab. Dazu tropft man rasch 800 ml einer einmolaren Lösung von Dimethylaluminiumchlorid in Hexan (800 mmol). Nach 10 Minuten Rühren gießt man die Lösung vorsichtig in 6 Liter Wasser ein, rührt die Mischung 30 Minuten und gibt dann die Lösung von 240 g Weinsäure in 1,6 Liter Wasser zu. Nach 30 Minuten macht man mit 450 ml konz. Ammoniaklösung alkalisch, trennt die organische Phase ab, extrahiert die wässrige Phase dreimal mit Dichlormethan, trocknet alle organischen Phasen mit Natrimsulfat und dampft das Lösungsmittel ab. Der Rückstand wird an Kieselgel mit Hexan/Aceton chromatographiert und aus Essigester/Diisopropylether kristallisiert, Ausbeute 17,2 g (35 % der Theorie), $[\alpha]_D$ = + 20 ° (0,5 % in Chloroform).

B) 8-Acetoxymethyl-8,9-didehydro-2-trimethylacetoxymethyl-6-ergolin-carbonitril

10,1 g 8-Acetoxy-8,9-didehydro-2-hydroxymethyl-6-ergolincarbonitril (3 mmol) löst man in 156 ml Pyridin und 30 ml Pivalinsäurechlorid und rührt 30 Minuten bei Raumtemperatur. Dann gibt man Eis zu, rührt wieder 30 Minuten, verdünnt mit Wasser, macht mit Ammoniak alkalisch und extrahiert mit Dichlormethan. Die organischen Phasen werden getrocknet, eingedampft und an Aluminiumoxid mit Dichlormethan chromatographiert. Durch Kristallisation aus Diisopropylether erhält man 8,9 g (70 % der Theorie), $[\alpha]_D$ = + 16 ° (0,5 % in Chloroform).

C) 8,9-Didehydro-8-hydroxymethyl-2-trimethylacetoxymethyl-6-ergolin-carbonnitril

In 100 ml Methanol suspendiert man 4,2 g 8-Acetoxymethyl-8,9-didehydro-2-trimethylacetoxymethyl-6-ergolincarbonitril (10 mmol), gibt 100 mg Natriummethylat zu und rührt eine Stunde bei Raumtemperatur. Man verdünnt unter Eiskühlung mit Wasser und saugt den Niederschlag ab, Ausbeute 3,65 g (96 % der Theorie), $[\alpha]_D$ = + 31 ° (0,5 % in Chloroform).

D) 8,9-Didehydro-8-hydroxymethyl-2-methyl-ergolin

Man kondensiert 25 ml trockenen Ammoniak in einem Kolben unter einer Argonatmosphäre, gibt 0,7 ml Anilin (7,5 mmol) und 920 mg Lithiumamid (40 mmol) zu und rührt 15 Minuten bei - 45 °C. Dann werden 1,89 g 8,9-Didehydro-8-hydroxymethyl-2-trimethylacetoxymethyl-6-ergolincarbonitril (5 mmol) zugegeben und 30 Minuten bei - 45 °C gerührt. Anschließend wird auf - 70 °C abgekühlt, mit 290 mg Lithium versetzt und bis zur Ausbildung einer intensiven Blaufärbung gerührt. 5 Minuten danach fügt man so viel Ammoniumchlorid zu, daß die Färbung verschwindet, tropft etwas Wasser zu und dampft den Ammoniak ab. Beim weiteren Verdünnen mit Wasser unter Eiskühlung entsteht ein Niederschlag, der abgesaugt und getrocknet wird, Ausbeute 782 mg (56 % der Theorie), $[\alpha]_D$ = - 111 ° (0,5 % in Pyridin).

6.) **8,9-Didehydro-2,8-dimethyl-ergolin**

Aus 8,9-Didehydro-6,8-dimethyl-ergolin stellt man, wie im vorstehenden Beispiel beschrieben, durch Prinsreaktion (Stufe A) 8,9-Didehydro-6,8-dimethyl-2-hydroxymethyl-ergolin her, Ausbeute nach Kristallisation aus Dichlormethan 67 %, $[\alpha]_D$ = - 183 ° (0,5 % in Pyridin). Durch Acylierung (Stufe B) mit Trimethylacetylchlorid erhält man 8,9-Didehydro-6,8-dimethyl-2-trimethylacetoxymethyl-ergolin in 80 % Ausbeute, $[\alpha]_D$ = - 147 ° (0,5 % in Chloroform).

Aus diesem wird durch Reduktion mit Lithium in Ammoniak (Stufe D) 8,9-Didehydro-2,6,8-trimethyl-ergolin in 95 % Ausbeute erhalten, $[\alpha]_D$ = - 179 ° (0,5 % in Chloroform). Anschließend wird mit Bromcyan (wie unter 1 beschrieben) das 8,9-Didehydro-2,8-dimethyl-6-ergolinylcarbonitril in 74 % Ausbeute dargestellt, $[\alpha]_D$ = + 36 ° (0,1 % in Chloroform).

5,4 g 8,9-Didehydro-2,8-dimethyl-6-ergolincarbonitril (20 mmol) werden in 1 Liter Eisessig gelöst mit 20 ml Wasser und 5 g Zinkstaub versetzt und 2 Stunden auf 110 °C erhitzt. Nach Zugabe von weiteren 3 g

Zinkstaub und 10 ml Wasser war nach 90 Minuten bei 110 °C die Reaktion beendet. Dann wird vom Niederschlag abfiltriert, das Filtrat weitgehend eingeengt, mit Ammoniaklösung alkalisch gemacht und mit Essigester ausgeschüttelt. Die organischen Phasen werden mit Natriumsulfat getrocknet und eingedampft, der Rückstand aus Essigester kristallisiert, Ausbeute 2,6 g (53 % der Theorie), $[\alpha]_D$ = - 141 ° (0,5 % in Chloroform).

7.) **Allgemeine Arbeitsvorschrift zur Alkylierung in 6-Stellung**

1,0 mmol Ergolin, 7,0 mmol Kaliumcarbonat, 0,5 mmol Tetrabutylammoniumhydrogensulfat und 50 ml Nitromethan werden in der angegebenen Reihenfolge zusammengegeben und mit 5 mmol Alkylierungsmittel versetzt. (Im Falle von Chlormethylcyclopropan verwendet man 10 Equivalente und gibt zusätzlich 2 Equivalente wasserfreies Lithiumiodid zu.) Man rührt bei 20 ° bis 50 ° C (zwischen 2 und 47 Stunden, DC) und filtriert anschließend über Sand/Celite. Der Filterrückstand wird gründlich mit Dichlormethan gewaschen und das Lösungsmittel im Vakuum entfernt. Der Rückstand wird über Aluminiumoxid (Akt. 3) filtriert (Essigester) und das nach Abziehen des Lösungsmittels erhaltene Rohprodukt kristallisiert. In manchen Fällen muß danach noch chromatographiert werden.

Analog werden hergestellt:

8,9-Didehydro-8-methyl-6-n-propyl-ergolin
Ausbeute: 64 % (kristallin aus Essigester), $[\alpha]_D$ = - 121,6 ° (c = 0,5 in Chloroform), Schmelzpunkt 127-130 °C (aus Essigester)

6-Allyl-8,9-didehydro-8-methyl-ergolin
Ausbeute: 15 % (kristallin aus Essigester/Hexan), $[\alpha]_D$ = - 161,5 ° (c = 0,4 in Chloroform), Schmelzpunkt 156-158 °C (aus Essigester/Hexan)

6-Cyclopropylmethyl-8,9-didehydro-8-methyl-ergolin
Ausbeute: 68 % (41 % kristallin aus Essigester/Hexan) $[\alpha]_D$ = -126,6 ° (c = 0,5 in Chloroform), Schmelzpunkt 192-195 °C (aus Essigester/Hexan)

8,9-Didehydro-6-ethyl-8-methyl-ergolin
Ausbeute: 30 % (25 % kristallin aus Essigester), Schmelzpunkt 225-229 °C subl. ab 180 °C (aus Essigester)

8.) **8,9-Didehydro-8-hydroxymethyl-6-n-propyl-ergolin**

Man löst 19 g 8,9-Didehydro-8-hydroxymethyl-ergolin (80 mmol) in 1,5 Liter DMF, gibt 46 ml Diazabicycloundecen und 73 ml 1-Brompropan (0,8 Mol) zu und rührt 5 Stunden bei Raumtemperatur und 1 Stunde bei 50 °C. Man läßt abkühlen, versetzt mit einer halbgesättigten Kochsalzlösung und extrahiert mit Dichlormethan. Die organischen Phasen werden getrocknet und, zuletzt nach Zugabe von Toluol, zur Trockene eingedampft. Der Rückstand wird an Kieselgel chromatographiert und aus Essigester kristallisiert, Ausbeute 13 g (58 % der Theorie), $[\alpha]_D$ = - 41 ° (0,5 % in Pyridin).

In analoger Weise wurden dargestellt:

6-Ethyl-8,9-didehydro-8-hydroxymethyl-ergolin
Ausbeute 43 %

6-Allyl-8,9-didehydro-8-hydroxymethyl-ergolin
Ausbeute 67 %

**Beispiel 1**

Analog der bei der Herstellung der Ausgangsverbindungen beschriebenen Arbeitsvorschrift (7) zur Alkylierung in 6-Stellung werden erhalten:

8,9-Didehydro-6-ethyl-2,8-dimethyl-ergolin
Ausbeute 15 %, $[\alpha]_D$ = - 184 ° (0,1 % in Chloroform)

8,9-Didehydro-2,8-dimethyl-6-n-propyl-ergolin
Ausbeute 60 %, $[\alpha]_D$ = - 152 ° (0,5 % in Chloroform)

6-Allyl-8,9-didehydro-2,8-dimethyl-ergolin
Ausbeute 18 %, $[\alpha]_D$ = - 192 ° (0,5 % in Chloroform)

6-Cyclopropylmethyl-8,9-didehydro-2,8-dimethyl-ergolin
Ausbeute 9 %, $[\alpha]_D$ = - 153 ° (0,1 % in Chloroform)

**Beispiel 2**

Analog der bei der Herstellung der Ausgangsverbindungen beschriebenen Methode (8) werden erhalten:
8,9-Didehydro-6-ethyl-8-hydroxymethyl-2-methyl-ergolin
Ausbeute 29 %, $[\alpha]_D$ = - 141 ° (0,5 % in Pyridin)
8,9-Didehydro-8-hydrdoxymethyl-2-methyl-6-n-propyl-ergolin
Ausbeute 13 %, $[\alpha]_D$ = - 78 ° (0,5 % in Pyridin)
6-Allyl-8,9-didehydro-8-hydroxymethyl-2-methyl-ergolin
Ausbeute 19 %, $[\alpha]_D$ = - 150 ° (0,5 % in Pyridin)
6-Cyclopropylmethyl-8,9-didehydro-8-hydroxymethyl-2-methyl-ergolin
Ausbeute 27 %

**Beispiel 3**

8-Acetoxymethyl-8,9-didehydro-2-methyl-6-n-propyl-ergolin

231 ml 8,9-Didehydro-8-hydroxymethyl-2-methyl-6-n-propyl-ergolin (0,79 mmol) löst man in 4 ml Pyridin, gibt 0,8 ml Acetanhydrid zu und rührt 30 Minuten bei Raumtemperatur. Dann gibt man Eis zu, läßt 1 Stunde stehen, macht mit Ammoniak alkalisch und extrahiert mit Dichlormethan. Die organischen Phasen werden mit Natriumsulfat getrocknet und eingedampft, Ausbeute nach Kristallisation aus Diisopropylether/Hexan 160 mg (60 % der Theorie), $[\alpha]_D$ = - 113 ° (0,5 % in Chloroform).

**Beispiel 4**

2-Chlor-8,9-didehydro-8-hydroxymethyl-6-n-propyl-ergolin

Man löst 0,97 g 8-Acetoxymethyl-8,9-didehydro-6-n-propyl-ergolin (3 mmol) in 900 ml Dioxan, gibt 1 g N-Chlorsuccinimid zu und rührt 16 Stunden bei 90 °C. Dann läßt man abkühlen, versetzt mit gesättigter Kochsalzlösung und schüttelt dreimal mit Essigester aus. Die organischen Phasen werden mit Natriumsulfat getrocknet, eingedampft und an Aluminiumoxid mit Dichlormethan und Essigester chromatographiert. Das Rohprodukt wird in 20 ml Tetrahydrofuran und 20 ml 1N Kalilauge gelöst und 4 Stunden bei Raumtemperatur gerührt. Es wird wie oben beschrieben aufgearbeitet, der Rückstand an Kieselgel mit Essigester/Methanol chromatographiert und schließlich aus Dichlormethan kristallisiert, Ausbeute 53 mg (6 % der Theorie), $[\alpha]_D$ = - 126 ° (0,1 % in Pyridin).

Analog werden die folgenden Verbindungen dargestellt:
2-Chlor-8,9-didehydro-8-methyl-6-n-propyl-ergolin
Ausbeute 27 %
2-Chlor-8,9-dideyhdro-6-ethyl-8-methyl-ergolin
Ausbeute 19 %
2-Chlor-8,9-didehydro-6-ethyl-8-hydroxymethyl-ergolin
Ausbeute 29 %

Die Ausgangsverbindung 8-Acetoxymethyl-8,9-didehydro-6-n-propyl-ergolin erhält man analog Beispiel 3, Ausbeute 52 %, $[\alpha]_D$ = - 88 ° (0,5 % in Chloroform).

**Beispiel 5**

2-Brom-8,9-didehydro-8-methyl-6-n-propyl-ergolin

533 mg 8,9-Didehydro-8-methyl-6-n-propyl-ergolin (2 mmol) werden in 40 ml Dioxan gelöst und mit 1,092 g Pyrrolidon-hydrotribromid (2,2 mmol) in 6 Portionen versetzt, die in Abständen von 15 Minuten zugegeben werden. Dazu gibt man 0,5 ml Aceton, rührt 15 Minuten nach und macht anschließend mit Ammoniak alkalisch. Die Mischung wird mit Wasser verdünnt und mit Dichlormethan ausgeschüttelt. Die organischen Phasen werden getrocknet und eingedampft, Der Rückstand wird an Kieselgel mit Hexan/Aceton chromatographiert und dann aus Essigester kristallisiert, Ausbeute 324 mg (47 % der Theorie), $[\alpha]_D$ = - 163 ° (0,5 % in Pyridin).

Analog werden dargestellt:
6-Allyl-2-brom-8,9-didehydro-8-methyl-ergolin
Ausbeute 47 %
2-Brom-8,9-didehydro-6-ethyl-8-hydroxymethyl-ergolin
Ausbeute 73 %
2-Brom-8,9-didehydro-8-hydroxymethyl-6-n-propyl-ergolin
Ausbeute 43 %, $[\alpha]_D$ = - 125 ° (0,5 % in Pyridin)

6-Allyl-2-brom-8,9-didehydro-8-hydroxymethyl-ergolin
Ausbeute 38 %

**Beispiel 6**
8,9-Didehydro-8-methyl-6-n-propyl-2-vinyl-ergolin
1. 8,9-Didehydro-8-methyl-2-morpholinomethyl-6-n-propyl-ergolin

7,47 g 8,9-Didehydro-8-methyl-6-n-propyl-ergolin, 24,0 g (0,19 mol) Morpholinhydrochlorid, 4,5 g (0,15 mol) Paraformaldehyd und 220 ml trockenes Dimethylformamid werden in der angegebenen Reihenfolge zusammengegeben und die Mischung in einem auf 100 °C vorgeheizten Olbad 30 Minuten gerührt. Zur Aufarbeitung gießt man die Reaktionslösung auf Eis, stellt mit 25 %iger $NH_3$-Lösung alkalisch und extrahiert mit Toluol. Das nach Trocknen über $Na_2SO_4$ und Abziehen des Toluols im Vakuum erhaltene Rohprodukt wird in 100 ml Trifluoressigsäure gelöst und 30 Minuten bei 60 °C gerührt. Danach gießt man die Reaktionsmischung auf Eis und stellt mit 25%iger $NH_3$-Lösung alkalisch. Nach Extraktion mit Dichlormethan, Trocknen über $Na_2SO_4$ und Abziehen des Lösungsmittels im Vakuum erhält man ein dunkles Ol, das durch Chromatographie gereinigt wird.
Ausbeute: 60 %, $[\alpha]_D$ = - 108,6 ° (c = 0,5 in Chloroform)

2. 8,9-Didehydro-2-formyl-8-methyl-6-n-propyl-ergolin

3,65 g dieser Mannichbase (10 mmol) werden in 160 ml Tetrahydrofuran p.A. gelöst, auf - 40 °C gekühlt und 1,6-3,2 ml (11,5-23,0 mmol) Triethylamin zugesetzt. Anschließend wird eine Lösung von 1,5 ml (12,3 mmol) tert.-Butylhypochlorid in 25 ml Tetrahydrofuran zügig zugetropft. Nach 5 Minuten wird die Mischung dünnschichtchromatographisch kontrolliert. Falls sich noch Edukt nachweisen läßt, wird noch Hypochlorid nachgegeben. 30 Minuten nach der letzten Zugabe (DC) gießt man den Ansatz auf Eis, alkalisiert mit 25%iger $NH_3$-Lösung und extrahiert mit Essigester. Das nach Abziehen des Lösungsmittels erhaltene Rohprodukt muß chromatographiert werden.
Ausbeute: 38 %, $[\alpha]_D$ = - 223,8 ° (c = 0,1 in Chloroform)

3. 8,9-Didehydro-2-(1-hydroxyethyl)-8-methyl-6-n-propyl-ergolin

1,47 g Aldehyd (5,0 mmol) werden in 150 ml trockenem Tetrahydrofuran gelöst und auf - 65 °C gekühlt. Es werden 10-15 mmol Methyl-Lithium (1,6M Lösung in Ether) in 5-7 Portionen zugegeben. Nach der letzten Zugabe läßt man auftauen und rührt noch 30 Minuten bei Raumtemperatur. Der Reaktionsverlauf wird dünnschichtchromatographisch verfolgt. Zur Aufarbeitung gießt man auf Eis, alkalisiert mit 25%iger $NH_3$-Lösung und extrahiert mit Essigester. Das nach Abziehen des Lösungsmittel erhaltene Rohprodukt wird chromatographiert.
Ausbeute: 63 %.

4. 8,9-Didehydro-8-methyl-6-n-propyl-2-vinyl-ergolin

300 mg des vorstehenden Rohproduktes werden in 40 ml Tetrahydrofuran p.A. gelöst und mit 1,4 ml (10 mmol) Triethylamin versetzt. Nach Zugabe von 0,8 (10 mmol) Methansulfonsäurechlorid rührt man noch 30 Minuten bei Raumtemperatur (DC nach 5 Minuten). Anschließend gießt man die Mischung auf Eis, alkalisiert mit 25%iger $NH_3$-Lösung und extrahiert mit Essigester. Das nach Abdampfen des Lösungsmittels im Vakuum erhaltene Rohprodukt wird chromatographiert.
Ausbeute nach Chromatographie (Essigester/Dichlormethan 1:1): 41 % (12 % kristallin aus Essigester/Hexan), Schmelzpunkt 172-174,5 °C (Zers.), $[\alpha]_D$ = - 201,3 ° (c = 0,2 in Chloroform).

**Beispiel 7**
8,9-Didehydro-8-hydroxymethyl-6-n-propyl-2-vinyl-ergolin
1. 8-Acetoxymethyl-8,9-didehydro-2-hydroxymethyl-6-n-propyl-ergolin

Wie im vorstehenden Beispiel als Stufe 1 beschrieben, erhält man aus 5,99 g 8-Acetoxymethyl-8,9-didehydro-6-n-propyl-ergolin (18,5 mmol) nach Aufarbeitung und Chromatographie 3,86 g der 2-Hydroxymethyl-Verbindung (etwa 56 % der Theorie) als Ol.

2. 8-Acetoxymethyl-8,9-didehydro-2-formyl-6-n-propyl-ergolin

Dieses Produkt wird in 275 ml Dichlormethan gelöst, mit 12,6 g Braunstein versetzt und 5 Stunden bei Raumtemperatur gerührt. Dann wird an Aluminiumoxid mit Dichlormethan chromatographiert und eingedampft, Ausbeute 1,98 g.

3. 8-Acetoxymethyl-8,9-didehydro-6-n-propyl-2-vinyl-ergolin

9,64 g Methyl-triphenylphosphoniumbromid werden in 160 ml wasserfreiem Tetrahydrofuran suspendiert und unter Eiskühlung mit 2,42 g Kalilum-tert.-butylat versetzt. Man rührt 30 Minuten unter Eiskühlung, senkt dann die Temperatur auf - 50 °C und tropft die Lösung des Aldehyds in 80 ml Tetrahydrofuran zu. Innerhalb von 3 Stunden läßt man auf - 10 °C aufwärmen, versetzt dann mit gesättigter Kochsalzlösung und schüttelt mit Essigester aus. Das Produkt wird an Kieselgel mit Hexan/Aceton chromatographiert, Ausbeute 1,48 g.

4. 8,9-Didehydro-8-hydroxymethyl-6-n-propyl-2-vinyl-ergolin

Die Verseifung der Acetylgruppe wird durch Lösen der Substanz in 40 ml Dichlormethan und 40 ml Methanol in Anwesenheit von 80 mg Natriummethylat in 22 Stunden bei Raumtemperatur erreicht. Die Mischung wird in eine gesättigte Kochsalzlösung eingegossen, mit Dichlormethan extrahiert und die organischen Phasen mit Natriumsulfat getrocknet und eingedampft. Der Rückstand wird an Aluminiumoxid mit Essigester chromatographiert, Ausbeute 527 mg, wovon aus Essigester 133 mg kristallisieren (Gesamtausbeute 2,3 %), $[\alpha]_D$ = - 141 ° (0,1 % in Pyridin).

**Beispiel 8**

8,9-Didehydro-2-ethyl-8-hydroxymethyl-6-n-propyl-ergolin

Wie bei der Herstellung der Ausgangsverbindungen und 5D beschrieben (Anilin ist nicht nötig) werden 370 mg 8,9-Didehydro-8-hydroxymethyl-6-n-propyl-2vinyl-ergolin (1,2 mmol) mit Lithium in Ammoniak reduziert, aufgearbeitet, an Kieselgel mit Essigester/Methanol chromatographiert und aus Dichlormethan/Diisopropylether/Hexan kristallisiert, Ausbeute 108 mg (29 % der Theorie, $[\alpha]_D$ = - 117 ° (0,1 % in Pyridin).

**Beispiel 9**

8,9-Didehydro-2-ethyl-8-methyl-6-n-propyl-ergolin

2,0 mmol Didehydro-2-(1-hydroxyethyl)-8-methyl-6-n-propyl-ergolin werden in 25 ml Eisessig gelöst und unter Argon 1,0 g (26,4 mmol) Natriumborhydrid (halbe Tabletten) zugegeben, Nach Ende der Reaktion (DC) gießt man auf Eis, alkalisiert unter Kühlung mit 25%iger $NH_3$-Lösung und extrahiert mit Dichlormethan. Das nach Abziehen des Lösungsmittels im Vakuum erhaltene Rohprodukt wird über Aluminiumoxid mit Dichlormethan chromatographiert.

Ausbeute: 65 % (52 % kristallin aus Essigester/Diisopropylether), Schmelzpunkt 152-154 °C, $[\alpha]_D$ = - 149,7 ° (c = 0,5 in Chloroform)

## Ansprüche

1.) Ergolin-Derivate der Formel I und deren Säureadditionssalze

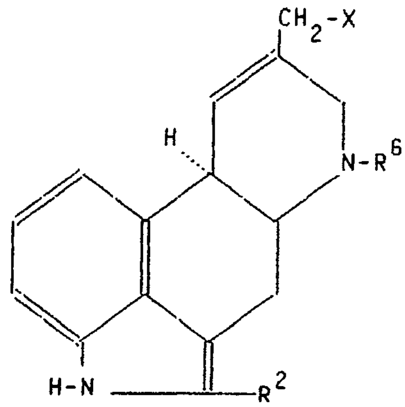

I

worin
$R^2$ Halogen, $C_{1-4}$-Alkyl, $C_{2-4}$-Alkenyl,
$R^6$ $C_{2-6}$-Alkyl, $C_{3-6}$-Alkenyl oder $C_{3-5}$-Cycloalkyl-$C_{1-2}$-Alkyl und
X Wasserstoff, Hydroxy oder $C_{1-6}$-Acyloxy darstellen.

2.) 8,9-Didehydro-2,8-dimethyl-6-n-propyl-ergolin
8,9-Didehydro-8-hydroxymethyl-2-methyl-6-n-propyl-ergolin
2-Brom-8,9-didehydro-8-methyl-6-n-propyl-ergolin
8,9-Didehydro-8-methyl-6-n-propyl-2-vinyl-ergolin
8,9-Didehydro-2-ethyl-8-methyl-6-n-propyl-ergolin

3.) Arzneimittel auf Basis der Verbindungen nach Anspruch 1-2.

4.) Verfahren zur Herstellung der Verbindungen nach Anspruch 1, dadurch, daß man

a) Verbindungen der Formel II

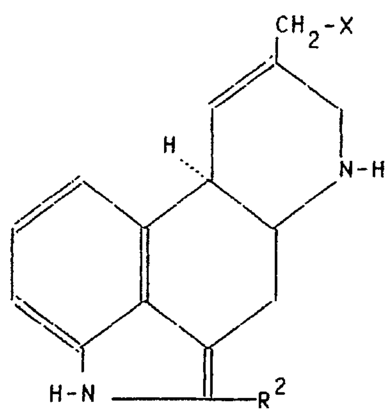

II

worin $R^2$ und X die obige Bedeutung haben, alkyliert oder alkenyliert zu Verbindungen mit $R^6$ in der obigen Bedeutung oder

b) Verbindungen der Formel III

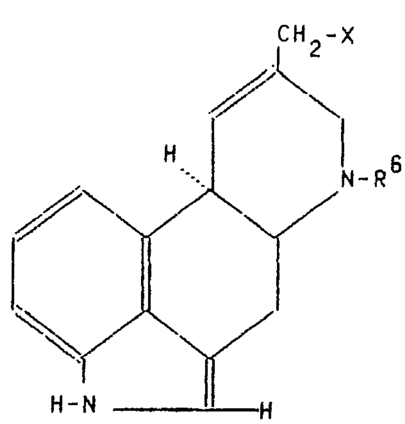

III

worin $R^6$ und X die obige Bedeutung haben, halogeniert zu Verbindungen der Formel I mit $R^2$ in der Bedeutung von Halogen oder

c) Verbindungen der Formel IV

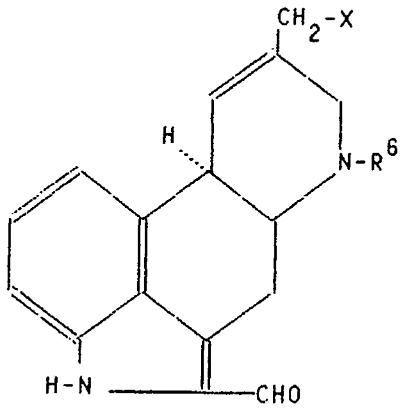

IV

worin $R^6$ und X die obige Bedeutung haben,

α) in einer Wittig-Reaktion zu Verbindungen mit $R^2$ in der Bedeutung eines $C_{2-4}$-Alkenylrestes umsetzt oder

β) in einen 1-Hydroxy-$C_{1-4}$-Alkylrest überführt und diesen gewünschtenfalls anschließend dehydratisiert zu Verbindungen mit $R^2$ in der Bedeutung eines $C_{2-4}$-Alkenylrestes und gewünschtenfalls anschließend die

nach α) oder β) erhaltenen Verbindungen, gegebenenfalls nach Veresterung des 2-$CH_2OH$-Restes, reduziert zu Verbindungen mit $R^2$ in der Bedeutung eines $C_{1-4}$-Alkylrestes und gewünschtenfalls anschließend die Acylgruppe in 8-Stellung abspaltet, die Isomeren trennt, und die Säureadditionssalze bildet.

5.) Verbindungen der Formel IV′

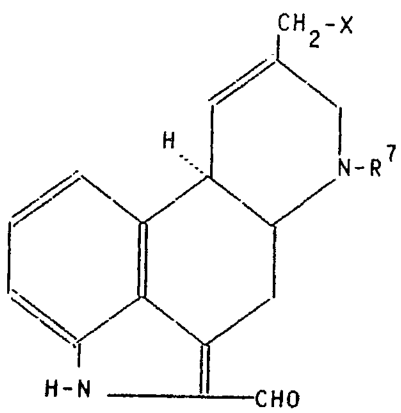

IV′

worin $R^7$ Carbonitril oder $R^6$ ist und $R^6$ und X die obige Bedeutung haben.

6.) Verfahren zur Herstellung der Zwischenverbindungen der Formel IV′ nach Anspruch 5 dadurch, daß man Verbindungen der Formel V

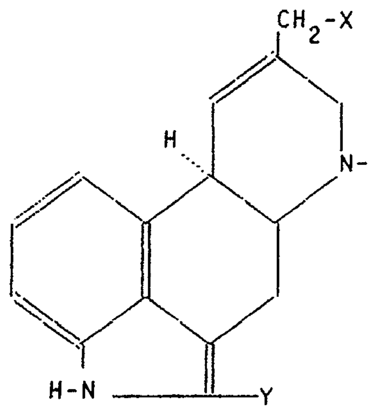

V

worin $R^7$ und X die obige Bedeutung haben und Y $CH_2$-OH oder

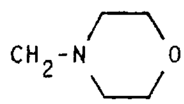 ist,

ist, oxidiert.

Europäisches Patentamt

# EUROPÄISCHER RECHERCHENBERICHT

Nummer der Anmeldung

EP 90250287.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| X | DD - A1 - 237 837 (AKADEMIE DER WISSENSCHAFTEN DER DDR) * Seiten 1,2; insbesondere Anspruch 1 * -- | 1-4 | C 07 D 457/02<br>A 61 K 31/48 |
| X | EP - A2/A3 - 0 160 842 (SCHERING AKTIENGESELLSCHAFT) * Ansprüche 1,9,10 * -- | 1-5 | |
| A | EP - A2/A3 - 0 030 351 (FARMITALIA CARLO ERBA S.D.A.) * Ansprüche 1,5 * -- | 1-3,5 | |
| A | EP - A1 - 0 003 667 (ELI LILLY AND COMPANY) * Ansprüche 1,5,6 * -- | 1-4 | |
| A | EP - A2 - 0 286 575 (SCHERING AKTIENGESELLSCHAFT) * Ansprüche 1,4 * ---- | 1-3 | |

RECHERCHIERTE SACHGEBIETE (Int Cl 5)

C 07 D 457/00

Der vorliegende Recherchenbericht wurde für alle Patentansprüche erstellt.

| Recherchenort | Abschlußdatum der Recherche | Prüfer |
|---|---|---|
| WIEN | 23-01-1991 | PETROUSEK |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82